# EUROPEAN PATENT APPLICATION

(11) **EP 1 384 482 A1**
(43) Date of publication of application: **28.01.2004**
(21) Application number: 02705098.8
(22) Date of filing: 11.03.2002
(51) Int. Cl.: A61K 31/7008, A61K 31/07, A61K 31/203, A61K 31/00, A61K 31/40, A61K 31/42, A61P 17/16

(54) **EXTERNAL SKIN COMPOSITIONS**

(30) Priority: 27.03.2001 JP 2001089314
(71) Applicant: Kanebo, Limited, Sumida-ku, Tokyo 131-0031 (JP)
(72) Inventor: SAYO, Tetsuya, Odawara-shi, Kanagawa 250-0863 (JP); SAKAI, Shingo, Odawara-shi, Kanagawa 250-0055 (JP); INOUE, Shintaro, Odawara-shi, Kanagawa 250-0851 (JP)
(74) Representative: VOSSIUS & PARTNER
(86) International application number: PCT/JP2002/002271
(87) International publication number: WO 2002/076470

(57) **Abstract**

The external skin care composition of the present invention comprises N-acetylglucosamine and at least one member selected from the group consisting of retinoid and pro-vitamin A.

The external skin care composition of the present invention has an effect of promoting the production of epidermal hyaluronic acid and can retain firmness and moisture of the skin.

## Description

### TECHNICAL FIELD

The present invention relates to an external skin care composition which can prevent or improve wrinkled skin, dry skin, tanned skin and aged skin while maintaining firmness and moisture of the skin and, more particularly, to an external skin care composition containing N-acetylglucosamine and at least one member selected from the group consisting of retinoid and pro-vitamin A.

### BACKGROUND ART

Hyaluronic acid has various functions such as retention of moisture in intercellular spaces, retention of cell structures by formation of a jelly-like matrix, retention of humidity and elasticity of the skin, resistance to an external force such as mechanical disorder, and prevention of bacterial infection (BIO INDUSTRY, Vol. 8, page 346, 1991).

It has been reported that the intensity of the staining signal of hyaluronic acid in the epidermis is reduced with aging (J. Invest. Dermatol., 102, 385, 1994), and that hyaluronic acid at solar elastosis site under irradiation with ultraviolet light is scarcely detected (Clin. Dermatol, (special number 5) 51, 53, 1997; Nagoya Med. J., 41, 27, 1997), thus causing dry skin and deterioration of firmness and elasticity of the skin, resulting in increase of wrinkles. To improve these skin conditions, a method of retaining moisture on the surface of the skin by applying a cosmetic composition containing hyaluronic acid formulated therein has been employed. However, since hyaluronic acid, as a large polymer, can not penetrate into the skin, a drastic improvement can not be expected. Therefore, it is expected to develop a substance capable of drastically improving cutaneous functions by promoting a cellular ability of production of hyaluronic acid.

As a substance capable of promoting the production of hyaluronic acid in epidermis, retinoic acid has been known so far. Retinoic acid is an essential substance which intrinsically exists in the epidermis and plays an important roles in the growth and differentiation of epidermal cells. Retinoic acid has widely used as an agent for restoring skin characteristics and an agent for reintegration of the skin in foreign countries in order to treat various dermatopathies, for example, acne vulgaris, fine wrinkles, psoriasis and age spots.

Various reports with respect to the effect of retinoic acid on (photo)aging have been made and its improving effect on the formation of fine wrinkles is recognized (Plastic Surgery, 42: 801, 1999; J. Dematol., 122, 91, 1990). Also it has been reported that deposition of mucopolysaccharides such as hyaluronic acid increases and the histological change of the photoaged skin is improved by applying retinoic acid (J. Dermatol. Sci., 11, 177, 1996). Therefore, it is considered that the deposition of hyaluronic acid, as an epidermal matrix component, and an increase in moisture achieved thereby may contribute remarkably to the effect of smoothing the skin surface of retinoic acid (The Japanese Journal of Dermatology, Vol. 110, No. 12, 1878, 2000) and an epidermal hyaluronio acid production promoting ingredient is useful for anti-wrinkling (prevention of formation of wrinkles or improvement of wrinkles) (FRAGRANCE JOURNAL, 4, 49, 1998).

However, retinoic acid causes skin irritation and it is required to formulate an external preparation containing low-concentration retinoic acid in order to prevent skin irritation. On the other hand, retinol or retinyl ester with less irritation must be metabolized in vivo into retinoic acid, as an activator, and it has exerts a smaller effect as compared with retinoic acid when the skin is benefitted. Therefore, it has been required to develop an external skin care ingredient which does not cause side effect such as skin irritation while maintaining the effect of retinoic acid. The present invention is based on such finding that a combination ofa retinoid and N-acetylglucosamine gives a synergistic improvement in the synthesis of hyaluronic acid of keratinocytes (epidermal cells).

Under these circumstances, an object of the present invention is to provide an external skin care ingredient which exerts a synergistic effect of promoting the production of hyaluronic acid by using in combination with a retinoid.

### DISCLOSURE OF THE INVENTION

In order to achieve the object described above, the present inventors have studied about an ability of promoting the hyaluronic acid production in various substances and found that N-acetylglucosamine acts synergically in combination with retinoids and also found that it exerts noticeably excellent effect of promoting the hyaluronic acid production by using in combination with pro-vitamin A. Thus, the present invention has been completed based on these findings.

Therefore, the present invention is directed to an external skin care composition containing N-acetylglucosamine and at least one member selected from the group consisting of a retinoid and pro-vitamin A.

### BRIEF DESCRIPTION OF THE DRAWINGS

Fig. 1 is a graph showing a synergistic effect of promoting the production of hyaluronic acid in human keratinocytes by using N-acetylglucosamine in combination with various retinoids.
Fig. 2 is a graph showing a synergistic effect of promoting the hyaluronic acid production in human keratinocytes by using N-acetylglucosamine in combination with β-carotene.

### BEST MODE FOR CARRYING OUT THE INVENTION

In the present invention, the external skin care composition generally refers to all compositions to be applied onto the skin including scalp, and includes medicaments, quasi-drugs, cosmetic compositions, bath medicines, hair growth promoters and scalp tonics.

According to the present invention, by formulating an effective amount of N-acetylglucosamine into the external skin care composition containing a retinoid, performances of the composition is substantially improved. Alternatively, it is possible to impart the same performances of a composition containing a high level of a retinoid to the composition by a combination of a low level (low concentration) of a retinoid and N-acetylglucosamine. Also it is possible to exert an excellent effect of promoting the production of hyaluronic acid by using in combination with pro-vitamin A which merely exerts a slight effect of promoting the hyaluronic acid production alone.

N-acetylglucosamine as the first essential ingredient of the present invention includes, but is not limited to, synthetic and fermentation products, and decomposition products obtained by decomposing chitin of crab, prawn or the like.

The amount of N-acetylglucosamine to be formulated into the external skin care composition is preferably controlled within a range from 0.001 to 10% by weight (hereinafter merely referred to as %), and particularly preferably from 0.01 to 5%, based on the total amount of the composition.

A retinoid as the second essential ingredient of the present invention, includes retinoio acid, retinal, retinol and fatty acid retinyl ester; and dehydroretinol, dehydroretinol and fatty acid dehydroretinyl ester. Pro-vitamin A is a compound having a retinilidene residue in the molecule and specific examples thereof include α-carotene, β-carotene, cryptoxanthin and echinenone. Two or more members of a retinoid and pro-vitamin A may be used in combination. Among these, retinoic acid is particularly preferable in view of the effect.

Retinoic acid includes the following isomers of retinoic acid, for example, all-trans-retinoic acid, 13-cis-retinoic acid, 11-cis-retinoic acid, 9-cis-retinoic acid and 3,4-dehydro-retinoic acid. Among these, all-trans-retinoic acid and 13-cis-retinoic acid, which are widely used as a remedy for acne vulgaris and photoaging in foreign countries, are preferable.

Retinol include the following isomers of retinol, for example, all-trans-retinol, 13-cis-retinol, 11-cis-retinol, 9-cis-retinol and 3,4-dehydro-retinol. Among these, all-trans-retinol and 13-cis-retinol are preferably because they are widely put on the market.

The fatty acid retinyl ester is a fatty acid ester of retinol. The fatty acid retinyl ester includes, but is not limited to, retinyl palmitate, retinyl formate, retinyl acetate, retinyl propionate, butyric acidretinyl, retinyl valerate, retinyl isovalerate, retinyl hexanoate, retinyl heptanoate, retinyl octanoate, retinyl nonanoate, retinyl decanoate, retinyl undecanoate, retinyl laurate, retinyl tridecanoate, retinyl myristate , retinyl pentadecanoate, retinyl heptadecanoate, stearic acidretinyl, isostearic acid ester, retinyl nonadecanoate, retinyl arachidonate, retinyl arachidonate, retinyl linoleate and retinyl oleate. The fatty acid moiety may be straight-chain or branched, or saturated or unsaturated.

As the fatty acid retinyl ester used in the present invention, commercially available retinyl palmitate, retinyl acetate and retinyl propionate are preferable.

The amount of the retinoid and/or pro-vitamin A to be formulated into the external skin care composition is preferably within a range from 0.0001 to 10%, and more preferably from 0.01 to 1%, based on the total amount of the composition.

The external skin care composition of the present invention appropriately contain tar colors; silicone oils such as dimethylpolysiloxane, methylphenylpolysiloxane, and cyclic silicone; carotenoid pigments such as lutein, astaxanthin, and fucoxanthin; color pigments such as iron oxide; antiseptics such as paraben and phenoxyethanol; hydrocarbons such as paraffin and petrolatum; vegetable oils such as olive squalane, rice squalane, whole rise oil, jojoba oil, castor oil, safflower oil, olive oil, macadamia nuts oil, and sunflower oil; waxes such as beeswax, Japan wax, and carnauba wax; ester oils such as octyldodecyl myristate, cetyl palmitate, isostearyl isostearate, and isopropyl myristate; lower alcohols such as ethanol; higher alcohols such as cetanol, behenyl alcohol, stearyl alcohol, and long-chain branched aliphatic alcohol; sterols and derivatives, such as cholesterol, phytosterol, branched fatty acid cholesterol ester, and macadamia nuts fatty acid phytosteryl ester; processed oils such as hardened oil; higher fatty acids such as stearic acid, myristic acid, isostearic acid, oleic acid, iso type long-chain fatty acid, and anti-iso type long-chain fatty acid; terpenes such as limonene and hydrogenated bisabolol; triglycerides such as tricapryl glyceryl caprate, glyceryl 2-ethylhexanoate, triiso type long-chain fatty acid glyceryl, and glyceryl tripalmitate; anionic surfactants such as sodium cetyl sulfate and N-stearoyl-L-glutamate; nonionic surfactants such as polyoxyethylene alkyl ether, polyoxyethylene fatty acid ester, polyoxyethylene polyhydric alcohol fatty acid ester, polyoxyethylene hydrogenaged castor oil, polyhydric alcohol fatty acid ester, modified silicone (e.g. polyoxyethylene-modified silicone), polyglycerin fatty acid ester, and sucrose ester; cationic surfactants such as tetraalkylammonium salt; amphoteric surfactants such as betaine type, sulfobetaine type and sulfoamino acid type surfactants; natural surfactants such as lecithin, lysophosphatidylcholine, ceramide, and cerebroside; pigments such as titanium oxide and zinc oxide; antioxidants such as dibutylhydroxytoluene; mineral salts such as sodium chloride, magnesium chloride, sodium sulfate, potassium nitrate, sodium sulfate, sodium metasilicate, and calcium chloride; organic acids and salts thereof, such as sodium citrate, potassium acetate, sodium succinate, sodium aspartate, sodium lactate, dichloroacetic acid, mevalonic acid, and glycyrrhizinic acid; organic amines and salts thereof, such as ethanolamine hydrochloride, ammonium nitrate, arginine hydrochloride, diisopropylamine salt, urea, and decarboxycarnosine; chelating agents such as edetic acid; thickeners such as xanthan gum, carboxyvinyl polymer, carrageenan, pectin, alkyl-modified carboxyvinyl polymer, and agar; neutralizers such as potassium hydroxide, diisopropanolamine, and triethanolamine; ultraviolet absorbers such as hydroxymethoxybenzophenone sulfonate; polyhydric alcohols such as dipropylene glycol, maltitol 1,3-butylene glycol, glycerin, propylene glycol, sorbitol, diglycerin, and raffinose; various amino acids; vitamins such as ascorbic acid, biotin, and tocopherol; and vitamin derivatives such as ascorbic acid phosphate ester salt and tocopherol nicotinate, in addition to the ingredients described above, as far as the object of the present invention can be achieved.

Furthermore, the effect of preventing formation of wrinkles is more enhanced by appropriately f ormulating dermal hyaluronic acid production promoters, such as N-methyl-L-serine and yeast extract; hyaluronic acid depolymerization inhibitors such as Kuritake (Naematoloma sublateritium)extract, Kurokawa (Boletopsis Leucomelas) extract, Mokkin (Hibiscus syriacus) extract, gambir extract, and clove extract; differentiation promoters of keratinocytes such as diisopropylaminedichloroacetic acid, niacin, mevalonic acid, hot spring water, sodium metasilicate, and orange homogenaized fruit; and skin barrier enhancers such as β -hydroxy -γ-aminobutyric acid and mevalonic acid; as far as the object of the present invention can be achieved.

### Examples

The present invention will be described in detail by the following Test Examples and Formulation Examples which do not limit the present invention.

### Test Example 1 (Hyaluronic acid production test to human normal keratinocytes)

Human keratinocytes (manufactured by Kurabo Industries, Ltd.) were seeded in a 24-well plate, cultured to confluence in a growth medium, and then 5 mmol/L of N-acetylglucosamine. 0.0001% of retinyl palmitate, 0.0001% of retinyl palmitate + 5 mmol/L of N-acetylglucosamine, 1.0 imol/L of retinoic acid, or 1.0 imol/L of retinoic acid + 5 mmol/L of N-acetylglucosamine were added, respectively. 24 hours after the addition, hyaluronic acid released into the medium was determined. The determination of hyaluronic acid was conducted using a commercially available hyaluronic acid determination kit (manufactured by Chugai Diagnostics Science).

The effects of test substances were defined as percentage (%) of the amount of hyaluronic acid in a cultured medium without a test substance. The results are shown below.

| Test substances | Hyaluronic acid production promotion ratio (%±S.D.) |
|---|---|
| *5 mmol/L of N-acetylglucosamine | 155±11.8 |
| *0.0001% of retinyl palmitate | 123±16.4 |
| *0.0001% of retinyl palmitate + 5 | 264±65.6 |
| mmol/L of N-acetylglucosamine | |
| *1.0 imol/L of retinoic acid | 250±20.8 |
| *1.0 imol/L of retinoic acid + 5 | 632±89.7 |
| mmol/L of N-acetylglucosamine | |

By adding 5 mmol/L of N-acetylglucosamine to cultured keratinocytes, the production of hyaluronic acid was increased by 1.55 times as compared with the no-addition group. Consequently, it has been found that N-acetylglucosamine promotes the hyaluronic acid production in keratinocytes. By adding 0.0001% of retinyl palmitate alone, or retinoic acid whose effect of promoting the production of hyaluronic acid has already been known alone, the production of hyaluronic acid was increased by 1.23 times or 2.5 times as compared with the no-addition group. By simulatnesouly adding 0.0001% of retinyl palmitate and 5 mmol/L of N-acetylglucosamine to the culture, the production of hyaluronic acid was remarkably increased by 2.64 times as compared with the no-addition group, and thus the production of hyaluronic acid was increased to the level higher than that achieved by the effect of 1.0 imol/L of retinoic acid. By simultaneously adding 1.0 imol/L of retinoic acid and 5 mmol/L of N-acetylglucosamine, the production of hyaluronic acid was increased by 6.32 times as compared with the no-addition group. Consequently, a noticeable synergic effect of the both substances was recognized.

### Test Example 2 (Hyaluronic acid production test to human keratinocytes)

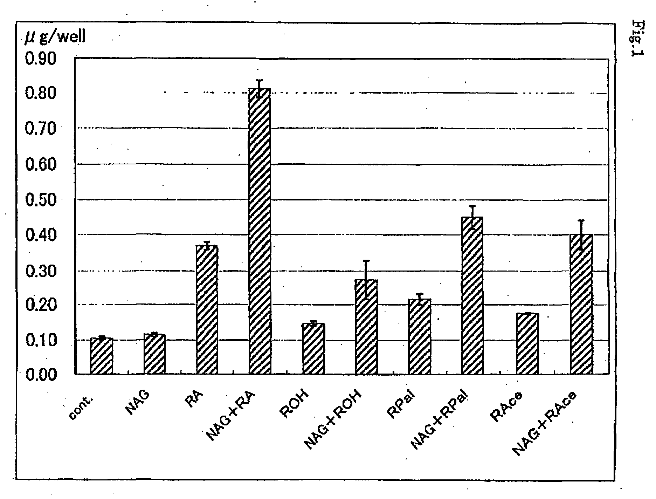

In the same manner as in Test Example 1, the test was conducted (n=3). As the test substance, 1 imol/L of retinoic acid (hereinafter referred to as RA), 1 imol/L of retinol (hereinafter referred to as ROH), 0.001% of retinyl palmitate (hereinafter referred to as RPa1) and 0.001% of retinyl acetate (hereinafter referred to as RAce) were used, and the determination was conducted with respect to the case where 5 mmol/L of N-acetylglucosamine (hereinafter referred to as NAG) and each of the above test substances were simultaneously added. The results are shown below and in Fig. 1.

| | ìg/well | S.D. |
|---|---|---|
| cont. (no addition) | 0.11 | 0.0043 |
| NAG | 0.12 | 0.0036 |
| RA | 0.37 | 0.0100 |
| NAG + RA | 0.81 | 0.0252 |
| ROH | 0.15 | 0.0061 |
| NAG + ROH | 0.27 | 0.0574 |
| RPal | 0.22 | 0.0156 |
| NAG + RPal | 0.45 | 0.0332 |
| RAce | 0.18 | 0.0016 |
| NAG + RAce | 0.40 | 0.0400 |

Retinoic acid-, retinal-, retinyl palmitate-, and retinyl acetate-addition groups increased the production of hyaluronic acid by 3.4, 1.4, 2.0 and 1.6 times, respectively, as compared with the no-addition group. By simultaneously adding together with N-acetylglucosamine, the production of hyaluronic acid was remarkably increased by 7.4, 2.5, 4.1 and 3.6 times, respectively. The addition of N-acetylglucosamine alone exerted lower effect (e.g. 1.1 times) as compared with the no-addition group. Therefore, it has been found that the production of hyaluronic acid is synergically promoted by using these retinosds in combination with N-acetylglucosamine.

### Test Example 3 (Hyaluronic acid production promotion test to human keratinocytes)

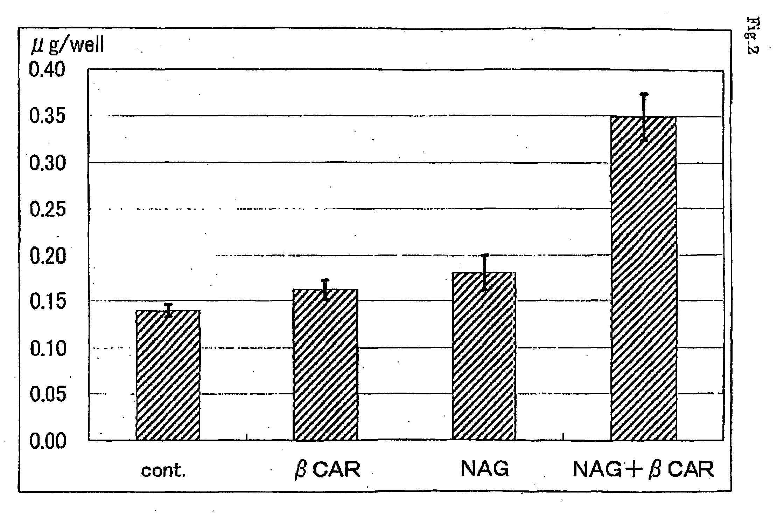

In the same manner as in Test Example 1, the test was conducted and the amount of hyaluronic acid was determined (n=3). As the test substance, 10 imol/L of β-carotene (hereinafter referred to as βCAR), NAG, and NAG + βCAR were used. The results are shown below and in Fig. 2.

| | ig/well | S.D. |
|---|---|---|
| cont. (no addition) | 0.14 | 0.007 |
| β CAR | 0.16 | 0.010 |
| NAG | 0.18 | 0.020 |
| NAG + βCAR | 0.35 | 0.025 |

N-acetylglucosamine and β-carotene increased the production of hyaluronic acid by 1.3 and 1.1 times as compared with the no-addition group. In case of simultaneously adding both substance, a remarkably high effect of promoting the production of hyaluronic acid (2.5 times) was exerted. Consequently, it has been found that the production of hyaluronic acid in epidermal cells is synergically promoted by using pro-vitamin A such as β-carotene in combination with N-acetylglucosamine.

Formulation Examples of the dermal hyaluronic acid production promoter of the present invention in various preparation forms will be described.

### Formulation Examples 1 to 3 (Skin creams)

According to the following formulation, N-acetylglucosamine and retinyl palmitate were formulated to prepare skin creams. All amounts are expressed by %.

### (1) Formulation

| | Formulation Example 1 | Formulation Example 2 | Formulation Example 3 |
|---|---|---|---|
| (A) | | | |
| Stearic acid | 1 | 1 | - |
| Isostearic acid | - | - | 1 |
| Glycerin monostearate | 2 | 2 | 2 |
| Behenyl alcohol | 2 | 2 | 2 |
| White beeswax | 1 | 1 | - |
| Cetyl myristate | 1 | 1 | 1 |
| Sorbitan sesquioleate | 1 | 1 | 1 |
| N-stearoylphytosphingosine | 0.1 | 0.1 | 0.1 |
| Hydrogenated lecithin | 0.1 | 0.1 | 0.1 |
| Vegetable squalane | 5 | 5 | 5 |
| Octyldodecyl myristate | 5 | 5 | 5 |
| Retinyl palmitate | 0.05 | 0.1 | 0.1 |

| (B) | | | |
|---|---|---|---|
| N-acetylglucosamine | 0.01 | 0.1 | 1.0 |
| 1,3-butylene glycol | 5 | 10 | 5 |
| Concentrated glycerin | 5 | 5 | 5 |
| Methyl paraoxybenzoate | 0.2 | 0.2 | 0.2 |
| Sodium ascorbyl phosphate | 0.2 | 0.2 | 0.2 |
| ester | | | |
| γ-aminobutyric acid | 0.1 | 0.1 | 0.1 |
| Sodium n-stearoylglutamate | 0.2 | 0.2 | 0.2 |
| Alkyl-modified | 0.05 | 0.05 | 0.005 |
| carboxyvinyl polymer | | | |
| Nicotinamide | 0.1 | 0.1 | 0.1 |
| Sarcosine | 0.1 | 0.1 | 0.1 |
| Purified water | balance | balance | balance |

### (2) Method of preparation

The ingredients (A) and (B) were dissolved while heating to 80ºC, mixed, cooled while stirring and then cooled to 30ºC to prepare skin creams.

### Formulation Examples 4 to 6 (Lotions)

According to the following formulation, N-acetylglucosamine and retinyl palmitate were formulated to prepare lotions.

### (1) Formulation

| | Formulation Example 4 | Formulation Example 5 | Formulation Example 6 |
|---|---|---|---|
| N-acetylglucosamine | 0.1 | 0.3 | 1.0 |
| Retinyl palmitate | 0.05 | 0.05 | 0.1 |
| 1,3-butylene glycol | 5 | - | 5 |
| Dipropylene glycol | - | 5 | 5 |
| Raffinose | 1 | 1 | 1 |
| Ethanol | - | - | 1 |
| Phenoxyethanol | 0.2 | 0.2 | 0.2 |
| Pectin | - | - | 0.05 |
| Xanthan gum | - | - | 0.1 |
| Sodium citrate | 0.05 | 0.05 | 0.05 |
| Field, horsetail extract (extracted with ethanol) | 0.1 | 0.1 | 0.1 |
| Diisopropylaminedichloroacetic acid | 0.2 | 0.2 | 0.2 |
| γ-amino-β-hydroxybutyric acid | 0.2 | 0.2 | 0.2 |
| Sodium hyaluronate | 0.001 | 0.001 | 0.001 |
| Dipotassium glycyrrhizinate | 0.2 | 0.2 | 0.2 |
| Kuritake (Naematoloma sublateritium) extract(extracted with ethanol) | 0.05 | 0.05 | 0.05 |
| Decaboxycarnosine hydrochloride | 0.05 | 0.05 | 0.05 |
| Perfume | 0.02 | 0.02 | 0.02 |
| Purified water | balance | balance | balance |

### (2) Method of preparation

The respective ingredients were dissolved while mixing and then stirred to prepare lotions.

### Formulation Examples 7 to 9 (Gels)

According to the following formulation, N-acetylglucosamine and retinyl palmitate were formulated to prepare gels.

### (1) Formulation

| | Formulation Example 7 | Formulation Example 8 | Formulation Example 9 |
|---|---|---|---|
| (A) | | | |
| Decamethylcyclopentasiloxane | 10 | 10 | 10 |
| Isostearyl isostearate | 1 | - | - |
| Olive oil | - | 1 | - |
| Macadamia nuts oil | - | - | 1 |
| Eucalyptus oil | 0.1 | - | 0.1 |
| Hexyldecanol | 1 | 0.1 | - |
| POE hydrogenated castor oil | 2 | 2 | 2 |
| (60E.O.) | | | |
| Spherical silicon powder | 1 | 1 | 5 |
| (Note 1) | | | |
| Retinyl palmitate | 0.05 | 0.05 | 0.05 |

| (B) | | | |
|---|---|---|---|
| N-acetylglucosamine | 0.1 | 0.1 | 0.1 |
| Glucosamine | - | 0.1 | - |
| Glucuronic acid | - | - | 0.1 |
| 1,3-butylene glycol | 5 | 10 | 5 |
| Sorbitol liquid | 3 | 3 | 3 |
| Polyethylene glycol 4000 | 1 | 1 | 1 |
| Carboxyvinyl polymer | 0.2 | 0.2 | 0.2 |
| Sugar ceramide (Note 2) | 0.1 | 0.1 | 0.1 |
| Methyl paraoxybenzoate | 0.2 | 0.2 | 0.2 |
| Mevalonolactone | 0.5 | 0.5 | 0.5 |
| Disodium edetate | 0.02 | 0.02 | 0.02 |
| Potassium hydroxide | 0.05 | 0.05 | 0.05 |
| Purified water | balance | balance | balance |
| Note 1: Tospearl 145A manufactured by GE TOSHIBA SILICONE CO.,LTD. | | | |
| Note 2: Bioceramide manufactured by Kibun Food Chemifa Co., Ltd. | | | |

### (2) Method of preparation

The ingredients (A) and (B) were dissolved while heating to 60ºC, mixed, cooled while stirring and then cooled to 30ºC to prepare gels.

### Formulation Example 10 to 12 (Lipophilic creams)

According to the following formulation, N-acetylglucosamine and retinyl palmitate were formulated to prepare lipophilic creams.

### (1) Formulation

| | Formulation Example 10 | Formulation Example 11 | Formulation Example 12 |
|---|---|---|---|
| (A) | | | |
| Co-modified silicon (Note 3) | 2 | 2 | 2 |
| POE-modified silicon dispersion | - | 2 | - |
| (Note 4) | | | |
| Squalane | - | - | 10 |
| Decamethylcyclopentasiloxane | 15 | 20 | 10 |
| Methylpolysiloxane | 5 | 2 | 3 |
| Long-chain branched fatty acid cholesteryl (Note 5) | - | - | 3 |
| Silicon elastomer dispersion (Note 6) | 5 | 2 | - |
| Retinyl palmitate | 0.1 | 0.1 | 0.1 |

| (B) | | | |
|---|---|---|---|
| N-acetylgluoosamine | 0.1 | 0.1 | 0.1 |
| Niacin | 0.1 | 0 | - |
| Kuritake (Naematoloma sublateritium) extract (extracted with ethanol) | - | 0.1 | - |
| Orange homogenized fruit | - | - | 0.1 |
| extract(Note 7) | | | |
| Sodium chloride | 1 | 1 | 1 |
| Dipropylene glycol | 5 | 5 | 5 |
| Concentrated glycerin | 5 | 5 | 5 |
| Raffinose | 1 | 1 | 1 |
| Methyl paraoxybenzoate | 0.3 | 0.3 | 0.3 |
| Glycyrrhiza extract (extracted with ethanol) | 0.1 | 0.1 | 0.1 |
| N-methyl-L-serine | 0.5 | 0.5 | 0.5 |
| Purified water | balance | balance | balance |
| Note 3: ABIL EM90 manufactured by Gold Schmidt Co. | | | |
| Note 4: Silicon BY22-008 manufactured by Dow Corning Toray Silicone Co., Ltd. | | | |
| Note 5: YOFCO CLE-NH manufactured by Nippon Fine Chemical Co., Ltd. | | | |
| Note 6: Torayfil manufactured by Dow Corning Toray Silicone Co., Ltd. | | | |
| Note 7: concentrated fruit juice manufactured by Koei Kogyo Co., Ltd. | | | |

### (2) Method of preparation

The ingredients (A) and (B) were dissolved with while heating to 60ºC, mixed, cooled while stirring and then cooled to 30ºC to prepare lipophilic creams.

### Formulation Examples 13 to 14 (Lotions)

According to the following formulation, lotions were prepared.

### (1) Formulation

| | Formulation Example 13 | Formulation Example 14 |
|---|---|---|
| N-acetylglucosamine | 0.1 | 0.1 |
| Retinyl palmitate | 0.1 | 0.2 |
| POE hydrogenated castor oil | 1.0 | 1.0 |
| (100E.O.) | | |
| Ethanol | 8.0 | 8.0 |
| 3-methyl-4-isopropylphenol | 0.1 | 0.1 |
| Polyethylene glycol | 1.0 | 1.0 |
| Dried orange peel extract | 0.1 | 0.1 |
| Lily extract | 0.1 | 0.1 |
| Orchid extract | 0.1 | 0.1 |
| Dipropylene glycol | 3.0 | 3.0 |
| Hydroxypropyl cellulose | 0.05 | 0.05 |
| Glycyrrhiza leave extract | 0.3 | 0.3 |
| d1-Camphor | 0.01 | - |
| Menthol | 0.02 | - |
| 1-Menthyl glyceryl ether | - | 0.1 |
| Purified water | balance | balance |

### (2) Method of preparation

The respective ingredients were dissolved while mixing and then stirred to prepare lotions.

### Formulation Example 15 (Gel)

According to the following formulation, a gel was prepared.

### (1) Formulation

| | Formulation Example 15 |
|---|---|
| (A) | |
| Retinyl palmitate | 0.1 |
| Decaglyceryl stearate | 1.0 |
| Dioctyl ether | 0.1 |
| Dioctyl carbonate | 0.1 |
| Dipropylene glycol | 3.0 |
| Octyl palmitate | 0.1 |
| Decaglyceryl isostearate | 0.5 |
| Eucalyptus oil | 0.01 |

| (B) | |
|---|---|
| N-acetylglucosamine | 0.1 |
| Carboxyvinyl polymer | 0.3 |
| Potassium hydroxide | 0.15 |
| Wisteria tea extract (Ampelosis | 0.2 |
| grossedentata extract) | |
| Marshmallow extract | 0.2 |
| Edelweiss extract | 0.5 |
| L-serine | 0.01 |
| Rasberryketone glucoside | 0.01 |
| Disodium edetate | 0.02 |
| Purified water | balance |

### (2) Method of preparation

The ingredients (A) and (B) were dissolved with heating to 64ºC, mixed, cooled while stirring and then cooled to 30ºC to prepare gels.

### Formulation Examples 16 and 17 (O/W emulsions)

According to the following formulation, O/W emulsions were prepared.

### (1) Formulation

| | Formulation Example 16 | Formulation Example 17 |
|---|---|---|
| (A) | | |
| Retinyl palmitate | 0.1 | 0.1 |
| Palmitic acid | 1.0 | 1.0 |
| Ceramide 2 | 0.01 | 0.01 |
| Carnauba wax | 1.0 | 1.0 |
| Cetyl palmitate | 1.0 | 1.0 |
| Macadamia nuts oil | 2.0 | 2.0 |
| Macadamia nuts oil fatty acid phytosteryl(Dihydrocholesteryl Macadamiate) | 0.5 | 0.5 |
| γ-orizanol | 0.05 | 0.05 |
| Phytosterol (GLYCINE SOJA | 0.1 | 0.1 |
| (SOYBEAN) STEROL) | | |
| Jojoba oil | 1.0 | 1.0 |
| Jojoba alcohol | 0.1 | 0.1 |
| Monoglyceryl hydroxystearate (Salacos MG, manufactured by Nishin Oil Co., Ltd.) | 0.3 | 0.3 |

| (B) | | |
|---|---|---|
| N-acetylglucosamine | 0.1 | 0.1 |
| Montmorillonite | 0.2 | 0.2 |
| Xanthan gum | 0.05 | 0.05 |
| Potassium N-stearoyl glutamate | 0.3 | 0.3 |
| Starch | 0.001 | 0.001 |
| Olive leaf extract | 0.1 | 0.1 |
| Maltitol liquid | 0.1 | 0.1 |
| Touchuukasou extract | 0.1 | 0.1 |
| (Cordyceps Sinensis Extract) | | |
| Hoelen extract | 0.1 | 0.1 |
| Kakyoku extract (Pyracantha Fortuneana Fruit Extract) | 0.1 | 0.1 |
| Ascorbic acid 2-glucoside (Ascorbyl Glucoside) | - | 1.0 |
| Potassium hydroxide | - | 0.2 |
| Purified water | balance | balance |

### (2) Method of preparation

After the ingredient (B) was mixed and heated to 80ºC, an oil phase obtained by melting the ingredient (A) while heating to 80ºC was added. Then, the mixture was emulsified while stirring using a homomixer to prepare O/W emulsions.

### INDUSTRIAL APPLICABILITY

As described above, the synergistic effect of promoting the hyaluronic acid production is exerted by using N-acetylglucosamine, a retinoid and/or pro-vitamin A in combination. By applying the external skin care composition of the present invention, the hyaluronic acid production, as a cell matrix ingredient, is promoted, thus making it possible to prevent aging of human skin (retention of firmness, elasticity and moisture of the skin). Therefore, the external skin care composition of the present invention is useful for use in medicaments, quasi-drugs, cosmetic compositions, bath medicines, hair growth promoters and scalp tonics.

## Claims

1. An external skin care composition comprising N-acetylglucosamine and at least one member selected from the group consisting of a retinoid and pro-vitamin A.

2. The external skin care composition according to claim 1, wherein said retinoid is at least one member selected from the group consisting of all-trans-retinol. 13-cis-retinol, all-trans-retinoic acid, 13-cis-retinoic acid, retinyl palmitate, retinyl acetate and retinyl propionate.

3. The external skin care composition according to claim 1, wherein said pro-vitamin A is selected from the group consisting of α-carotene, β-carotene, γ-carotene, cryptoxanthin and echinenone.

4. The external skin care composition according to any one of claims 1 to 3, which contains N-acetylglucosamine in the amount of 0.001 to 10% by weight based on the total amount of said composition and a retinoid and/or pro-vitamin A in the amount of 0.0001 to 10% by weight based on the total amount of said composition

5. The external skin care composition according to any one of claims 1 to 4, which is a cosmetic composition.

6. A makeup method for preventing or improving wrinkled skin, dry skin, tanned skin or aged skin, which comprises applying the external skin care composition according to any one of claims 1 to 5 onto the skin.
